# EUROPEAN PATENT APPLICATION

(11) **EP 4 814 119 A1**
(43) Date of publication of application: **30.09.2026**
(21) Application number: 24894247.6
(22) Date of filing: 29.01.2024
(51) Int. Cl.: C12N 15/77, C12P 13/10, C12P 13/08, C12P 13/14

(54) **CORYNEBACTERIUM SP. MICROORGANISM HAVING IMPROVED L-AMINO ACID PRODUCTIVITY AND METHOD FOR PRODUCING L-AMINO ACIDS USING SAME**

(30) Priority: 21.11.2023 KR 20230162390
(71) Applicant: Daesang Corporation, Seoul 03130 (KR)
(72) Inventor: RYU, Mi, Seoul 07789 (KR); HONG, In Pyo, Seoul 07789 (KR); LEE, Dong Seok, Seoul 07789 (KR); KANG, Dong Hun, Seoul 07789 (KR); YOON, Sun Jun, Seoul 07789 (KR); LEE, Jae Hoon, Seoul 07789 (KR); PARK, Hyun Jung, Seoul 07789 (KR)
(74) Representative: Ipsilon
(86) International application number: PCT/KR2024/001311
(87) International publication number: WO 2025/110347

(57) **Abstract**

The present invention relates to a Corynebacterium sp. microorganism having improved L-amino acid productivity and a method for producing L-amino acids using same. The Corynebacterium sp. microorganism can have an improved production yield of L-amino acids, compared to a parent strain, due to weakened activity or inactivity of a carotenoid biosynthesis enzyme.

## Description

### Technical Field

The present invention relates to a *Corynebacterium* sp. microorganism having improved L-amino acid productivity and a method of producing L-amino acid using the same.

### Background Art

Amino acids are the basic building blocks of proteins that make up the bodies of living organisms. They are classified into essential amino acids, which are not synthesized or are difficult to synthesize in the body, and thus must be consumed through food, and non-essential amino acids, which can be metabolically synthesized in the body. Naturally synthesized L-amino acids are used not only as amino acid fortifiers but also as raw materials in various applications, including food, health functional foods, pharmaceuticals, and cosmetics.

L-amino acids were industrially produced by fermentation methods using naturally occurring microorganisms or mutant microorganisms modified therefrom so as to have improved amino acid productivity. Recently, in order to improve the efficiency of production of L-amino acids, genetic recombination technology has been applied to microorganisms such as *Escherichia coli* and *Corynebacterium*. When genetic recombination technology is used, it is possible to increase amino acid productivity by increasing the activity of an enzyme involved in amino acid biosynthesis or by inhibiting feedback caused by produced L-amino acid. In addition, it is possible to improve amino acid productivity by regulating the expression of amino acid exporter genes. US Patent No. 5,972,663 describes artificially overexpressing genes such as *mex*, *bmr* and *qacA* in several strains (such as *E. coli*) to enhance the abilities of the strains to produce L-cysteine, L-cystine, N-acetylserine and thiazolidine derivatives. European Patent No. 1016710 describes artificially overexpressing the genes *yahN, yeaS, yfiK* and *yggA* in an *Escherichia* sp. strain to increase the ability of the strain to produce L-glutamic acid, L-lysine, L-threonine, L-alanine, L-histidine, L-proline, L-arginine, L-valine and L-isoleucine. Korean Patent No. 10-1023925 describes artificially overexpressing the gene *yddG* in an *Escherichia* sp. strain to increase the ability of the strain to produce L-tryptophan and L-phenylalanine.

However, various proteins such as enzymes, transcription factors, and transport proteins are involved in the biosynthetic pathways of amino acids, and thus much research is still needed to develop recombinant microorganisms or mutant strains having improved L-amino acid productivity.

### [Prior Art Documents]

### [Patent Documents]

(Patent Document 1) US Patent No. 5,972,663
(Patent Document 2) European Patent No. 1016710
(Patent Document 3) Korean Patent No. 10-1023925

### DISCLOSURE

### Technical Problem

An object of the present invention is to provide a *Corynebacterium sp. microorganism* having improved L-amino acid productivity.

Another object of the present invention is to provide a method of producing L-amino acid using the microorganism.

### Technical Solution

One aspect of the present invention provides a *Corynebacterium* sp. microorganism having improved L-amino acid productivity by having a weakened or inactivated activity of a carotenoid biosynthesis enzyme.

As used herein, the term "carotenoid biosynthesis enzyme" refers to an enzyme involved in the carotenoid biosynthesis pathway. *Corynebacterium glutamicum* contains glycosylated C50 carotenoid decaprenoxanthin as yellow pigment, which is synthesized starting from isopentenyl pyrophosphate, generated in the non-mevalonate pathway, via farnesyl pyrophosphate, geranylgeranyl pyrophosphate, lycopene, and flavuxanthin. It is known that genes such as crtE, crtB, crtB2, crtI, crtEb, and crtYe/f are involved in the carotenoid biosynthesis pathway.

The crtB gene encodes phytoene synthase and is a cluster containing the Cgl0626 (geranylgeranyl pyrophosphate synthase) gene, the Cgl0624 (phytoene/squalene synthetase) gene and the Cgl0623 (phytoene dehydrogenase and related proteins) gene.

The crtB2 gene encodes phytoene synthase and is a cluster containing the Cgl2433 (phytoene/squalene synthetase) gene, the Cgl2432 (phytoene dehydrogenase and related proteins) gene and the Cgl2431 (phytoene dehydrogenase and related proteins) gene.

The carotenoid biosynthesis enzyme in the present invention may be a polypeptide having the activity of phytoene synthase encoded by the crtB gene, the crtB2 gene, or the cluster of the crtB/crtB2 genes, without being limited thereto.

Information on the nucleic acid and protein sequences of the carotenoid biosynthesis enzyme is available from known sequence databases (e.g., GenBank, UniProt).

As used herein, the term "weakened activity" means that the expression level of a gene encoding a protein of interest, such as an enzyme, a transcription factor or a transport protein, has decreased compared to that in the parent microorganism, that is, a wild-type strain or a strain before modification. The term "weakened activity" includes: a case in which the activity of the protein itself has decreased compared to the activity of the protein, originally possessed by the microorganism, through substitution, insertion, deletion, or a combination thereof of one or more nucleotides in a nucleotide sequence encoding the gene; a case in which the overall activity of the protein in the cell is lower than that in the wild-type strain or the strain before modification due to decreased expression or translation of the gene encoding the protein; and a combination thereof.

According to one embodiment of the present invention, the weakening of the activity of the carotenoid biosynthesis enzyme may be achieved by insertion, substitution, deletion, or a combination thereof of all or part of the gene encoding the carotenoid biosyntheis enzyme.

As used herein, the term "inactivated" means that a gene encoding a protein such as an enzyme, a transcription factor or a transport protein is not expressed at all compared to that in the native strain, that is, the wild-type strain or the strain before modification, or that the gene has no activity even though it is expressed.

According to one embodiment of the present invention, the gene encoding the carotenoid biosynthesis enzyme may be at least one selected from the group consisting of Cgl0626, Cgl0624, Cgl0623, Cgl2433, Cgl2432 and Cgl2431.

According to one embodiment of the present invention, the Cgl0626 gene may comprise the nucleotide sequence of SEQ ID NO: 1 and encode the amino acid sequence of SEQ ID NO: 2. In addition, the Cgl0624 gene may comprise the nucleotide sequence of SEQ ID NO: 3 and encode the amino acid sequence of SEQ ID NO: 4. In addition, the Cgl0623 gene may comprise the nucleotide sequence of SEQ ID NO: 5 and encode the amino acid sequence of SEQ ID NO: 6. In addition, the Cgl2433 gene may comprise the nucleotide sequence of SEQ ID NO: 7 and may encode the amino acid sequence of SEQ ID NO: 8. In addition, the Cgl2432 gene may comprise the nucleotide sequence of SEQ ID NO: 9 and may encode the amino acid sequence of SEQ ID NO: 10. In addition, the Cgl2431 gene may comprise the nucleotide sequence of SEQ ID NO: 11 and may encode the amino acid sequence of SEQ ID NO: 12.

The nucleotide sequence or amino acid sequence of the carotenoid biosynthesis enzyme according to the present invention may comprise or essentially consist of a sequence having at least 70%, at least 80%, at least 90%, at least 98%, at least 99%, or at least 99.9% homology to the nucleotide sequence of SEQ ID NO: 1, 3, 5, 7, 9 or 11 or the amino acid sequence of SEQ ID NO: 2, 4, 6, 8, 10 or 12, and may retain its original function.

As used herein, the term "improved productivity" means that L-amino acid productivity has increased compared to that of the parent strain. The term "parent strain" refers to a wild-type strain or mutant strain to be mutated, and includes a strain that is to be mutated directly or to be transformed with a recombinant vector or the like. In the present invention, the parent strain may be a wild-type *Corynebacterium* sp. strain or a *Corynebacterium* sp. microorganism or strain mutated from the wild-type strain.

The *Corynebacterium* sp. microorganism may be one known in the art, and for example, may be, but is not limited to, *Corynebacterium glutamicum*, *Corynebacterium crudilactis*, *Corynebacterium deserti*, *Corynebacterium callunae*, *Corynebacterium suranareeae*, *Corynebacterium lubricantis*, *Corynebacterium doosanense*, *Corynebacterium efficiens*, *Corynebacterium uterequi*, *Corynebacterium stationis*, *Corynebacterium pacaense*, *Corynebacterium singulare*, *Corynebacterium humireducens*, *Corynebacterium marinum*, *Corynebacterium halotolerans*, *Corynebacterium spheniscorum*, *Corynebacterium freiburgense*, *Corynebacterium striatum*, *Corynebacterium canis*, *Corynebacterium ammoniagenes*, *Corynebacterium renale*, *Corynebacterium pollutisoli*, *Corynebacterium imitans*, *Corynebacterium caspium*, *Corynebacterium testudinoris*, *Corynebacaterium pseudopelargi,* or *Corynebacterium flavescens*.

According to one embodiment of the present invention, the *Corynebacterium* sp. microorganism may be *Corynebacterium glutamicum*.

The *Corynebacterium* sp. microorganism having improved L-amino acid productivity according to the present invention may have improved L-amino acid productivity by having a weakened or inactivated activity of the carotenoid biosynthesis enzyme.

According to one embodiment of the present invention, the *Corynebacterium* sp. microorganism having improved L-amino acid productivity may lack at least one selected from the group consisting of Cgl0626, Cgl0624, Cgl0623, Cgl2433, Cgl2432 and Cgl2431, which encode carotenoid biosynthesis enzymes.

Specifically, the *Corynebacterium* sp. microorganism having improved L-amino acid productivity exhibits increased L-amino acid productivity compared to the parent strain, and in particular, the amount of L-amino acid produced by the *Corynebacterium* sp. microorganism may be at least 1%, 5%, 10%, 15%, 20%, 25%, 30%, 35%, 40%, 45%, 50%, 55%, 60%, 65%, 70%, 75%, 80%, 85%, 90%, 95%, or 100% higher than that produced by the parent strain, or may be 1.1-fold, 1.5-fold, 2-fold, 2.5-fold, 3-fold, 3.5-fold, 4-fold, 4.5-fold, 5-fold, 5.5-fold, 6-fold, 6.5-fold, 7-fold, 7.5-fold, 8-fold, 8.5-fold, 9-fold, 9.5-fold, 10-fold, 20-fold, 30-fold, 40-fold, 50-fold, 60-fold, 70-fold, 80-fold, 90-fold, or 100-fold higher than that produced by the parent strain, without being limited thereto.

According to one embodiment of the present invention, the L-amino acid may be at least one selected from the group consisting of L-alanine, L-isoleucine, L-valine, L-leucine, L-methionine, L-asparagine, L-cysteine, L-glutamine, L-serine, L-threonine, L-phenylalanine, L-tryptophan, L-tyrosine, L-aspartic acid, L-glutamic acid, L-arginine, L-histidine, L-lysine, L-glycine, L-proline, and L-citrulline.

For example, the L-amino acid may be L-arginine, L-citrulline, L-lysine, or L-glutamine, without being limited thereto.

The *Corynebacterium* sp. microorganism according to one embodiment of the present invention may be constructed using a recombinant vector lacking a gene encoding the carotenoid biosynthetic enzyme in a parent strain.

As used in the present invention, the term "vector" refers to any type of nucleic acid sequence transfer structure that is used as a means for transferring and expressing a gene of interest in a host cell. Unless otherwise specified, the term "vector" may mean one allowing the nucleic acid sequence contained therein to be expressed after insertion into the host cell genome and/or one allowing the nucleic acid sequence to be expressed independently. This vector comprises essential regulatory elements operably linked so that an inserted gene can be expressed. As used herein, the term "operably linked" means that a gene of interest and regulatory sequences thereof are functionally linked together in a manner enabling gene expression, and the "regulatory elements" include a promoter sequence for initiating transcription, any operator sequence for regulating transcription, a sequence encoding suitable mRNA ribosome-binding sites, and a sequence for regulating termination of transcription and translation.

The vector that is used in the present invention is not particularly limited as long as it may replicate in a host cell, and any vector known in the art may be used. Examples of the vector include a natural or recombinant plasmid, cosmid, virus and bacteriophage. Examples of a phage vector or cosmid vector include, but are not limited to, pWE15, M13, λMBL3, λMBL4, λIXII, λASHII, λAPII, λt10, λt11, Charon4A, and Charon21A, and examples of a plasmid vector include, but are not limited to, pBR series, pUC series, pBluescriptII series, pGEM series, pTZ series, pCL series, and pET series.

The vector may typically be constructed as a vector for cloning or as a vector for expression. The vector for expression may be a conventional vector that is used in the art to express a foreign gene or protein in a plant, animal, or microorganism, and may be constructed through various methods known in the art.

The "recombinant vector" that is used in the present invention may be constructed using a prokaryotic or eukaryotic cell as a host, and may replicate regardless of the genome of the host cell or may be integrated into the genome itself. The host cell may contain a replication origin, which is a particular nucleotide sequence which enables the vector to replicate in the host cell and from which replication starts. For example, when the vector used is an expression vector and uses a prokaryotic cell as a host, the vector generally comprises a strong promoter capable of promoting transcription (e.g., pLλ promoter, CMV promoter, trp promoter, lac promoter, tac promoter, and T7 promoter), a ribosome binding site for initiation of translation, and a transcription/translation termination sequence. When a eukaryotic cell is used as a host, the vector comprises a replication origin operating in the eukaryotic cell, and examples of the replication origin include, but are not limited to, an f1 replication origin, an SV40 replication origin, a pMB1 replication origin, an adeno replication origin, an AAV replication origin, and a BBV replication origin. In addition, the vector may comprise a promoter derived from the genome of a mammalian cell (e.g., metallothionein promoter) or a promoter derived from a mammalian virus (e.g., adenovirus late promoter, vaccinia virus 7.5K promoter, SV40 promoter, cytomegalovirus promoter, and HSV-tk promoter), and generally has a polyadenylation sequence as a transcription termination sequence.

The recombinant vector may comprise a selection marker. The selection marker serves to select a transformant (host cell) transformed with the vector, and since only cells expressing the selection marker can survive in the medium treated with the selection marker, it is possible to select transformed cells. Representative examples of the selection marker include, but are not limited to, ampicillin, kanamycin, streptomycin, and chloramphenicol.

The transformant may be produced by inserting the recombinant vector into a host cell, and the transformant may be obtained by introducing the recombinant vector into an appropriate host cell. The host cell is a cell capable of stably and continuously cloning or expressing the expression vector, and any host cell known in the art may be used.

Where the vector is transformed into prokaryotic cells to generate recombinant microorganisms, examples of host cells that may be used include, but are not limited to, *E*. *coli* sp. strains such as *E. coli* DH5α, *E. coli* JM109, *E. coli* BL21, *E. coli* RR1, *E. coli* LE392, *E. coli* B, *E. coli* X 1776, *E. coli* W3110, and *E. coli* XL1-Blue, *Corynebacterium* sp. strains, *Bacillus* sp. strains such as *Bacillus subtilis* and *Bacillus thuringiensis,* and various Enterobacteriaceae strains such as *Salmonella typhimurium, Serratia marcescens,* and *Pseudomonas* species.

Where the vector is transformed into eukaryotic cells to generate recombinant microorganisms, examples of host cells that may be used include, but are not limited to, yeast (e.g., *Saccharomyces cerevisiae*), insect cells, and plant cells and animal cells, such as Sp2/0, CHO K1, CHO DG44, PER.C6, W138, BHK, COS7, 293, HepG2, Huh7, 3T3, RIN, and MDCK cell lines.

As used in the present invention, the term "transformation" refers to a phenomenon in which external DNA is introduced into a host cell, thereby artificially causing genetic changes, and the term "transformant" refers to a host cell into which external DNA has been introduced and in which the expression of the gene of interest is stably maintained.

The transformation may be performed using a suitable vector introduction technique selected depending on the host cell, so that the gene of interest or a recombinant vector comprising the same may be expressed in the host cell. For example, introduction of the vector may be performed by electroporation, heat-shock, calcium phosphate (CaPO₄) precipitation, calcium chloride (CaCl₂) precipitation, microinjection, polyethylene glycol (PEG) method, DEAE-dextran method, cationic liposome method, lithium acetate-DMSO method, or any combination thereof, without being limited thereto. As long as the transformed gene may be expressed in the host cell, it may be inserted into the chromosome of the host cell, or may exist extrachromosomally, without being limited thereto.

The transformant may include a cell transfected, transformed, or infected with the recombinant vector of the present invention *in vivo* or *in vitro,* and may be used interchangeably with the term "recombinant host cell", "recombinant cell", or "recombinant microorganism".

Genes inserted into the recombinant vector for transformation according to the present invention may be integrated into a host cell such as a *Corynebacterium* sp. microorganism by homologous recombination crossover.

According to one embodiment of the present invention, the host cell may be a *Corynebacterium* sp. microorganism. The *Corynebacterium* sp. microorganism may be, for example, *Corynebacterium glutamicum*.

Another aspect of the present invention provides a method for producing L-amino acid comprising steps of: culturing the *Corynebacterium* sp. microorganism in a medium; and recovering L-amino acid from the microorganism or the medium in which the microorganism has been cultured.

The culturing may be performed using a suitable medium and culture conditions known in the art, and any person skilled in the art may easily adjust and use the medium and the culture conditions. Specifically, the medium may be a liquid medium, without being limited thereto. Examples of the culturing method include, but are not limited to, batch culture, continuous culture, fed-batch culture, or a combination thereof.

According to one embodiment of the present invention, the medium should meet the requirements of a specific strain in a proper manner, and may be appropriately modified by a person skilled in the art. For culture media for *Corynebacterium* sp. microorganisms, reference may be made to, but not limited to, a known document (Manual of Methods for General Bacteriology, American Society for Bacteriology, Washington D.C., USA, 1981).

According to one embodiment of the present invention, the medium may contain various carbon sources, nitrogen sources, and trace element components. Examples of carbon sources that may be used include: sugars and carbohydrates such as glucose, sucrose, lactose, fructose, maltose, starch, and cellulose; oils and fats such as soybean oil, sunflower oil, castor oil, and coconut oil; fatty acids such as palmitic acid, stearic acid, and linoleic acid; alcohols such as glycerol and ethanol; and organic acids such as acetic acid. These substances may be used individually or as a mixture, without being limited thereto. Examples of nitrogen sources that may be used include peptone, yeast extract, meat extract, malt extract, corn steep liquor, soybean meal, urea, or inorganic compounds such as ammonium sulfate, ammonium chloride, ammonium phosphate, ammonium carbonate, and ammonium nitrate. The nitrogen sources may also be used individually or as a mixture, without being limited thereto. Examples of phosphorus sources that may be used include, but are not limited to, potassium dihydrogen phosphate or dipotassium hydrogen phosphate or the corresponding sodium-containing salts. In addition, the culture medium may contain, but is not limited to, metal salts such as magnesium sulfate or iron sulfate, which are required for growth. In addition, the culture medium may contain essential growth substances such as amino acids and vitamins. Moreover, suitable precursors may be used in the culture medium. The medium or individual components may be added to the culture medium batchwise or in a continuous manner by a suitable method during culturing, without being limited thereto.

According to one embodiment of the present invention, the pH of the culture medium may be adjusted by adding compounds such as ammonium hydroxide, potassium hydroxide, ammonia, phosphoric acid and sulfuric acid to the microorganism culture medium in an appropriate manner during the culturing. In addition, during the culturing, foaming may be suppressed using an anti-foaming agent such as a fatty acid polyglycol ester. Additionally, to keep the culture medium in an aerobic condition, oxygen or an oxygen-containing gas (for example, air) may be injected into the culture medium. The temperature of the culture medium may be generally 20°C to 45°C, for example, 25°C to 40°C. The culturing may be continued until a desired amount of a useful substance is produced. For example, the culturing time may be 10 hours to 160 hours.

According to one embodiment of the present invention, in the step of recovering L-amino acid from the cultured *Corynebacterium* sp. microorganism or the medium in which the microorganism has been cultured, the produced L-amino acid may be collected or recovered from the medium using a suitable method known in the art depending on the culture method. Examples of a method that may be used to recover the produced L-amino acid include, but are not limited to, centrifugation, filtration, extraction, spraying, drying, evaporation, precipitation, crystallization, electrophoresis, fractional dissolution (e.g., ammonium sulfate precipitation), chromatography (e.g., ion exchange, affinity, hydrophobicity and size exclusion), and the like.

According to one embodiment of the present invention, the step of recovering L-amino acid may be performed by centrifuging the culture medium at a low speed to remove biomass and separating the obtained supernatant through ion-exchange chromatography.

According to one embodiment of the present invention, the step of recovering L-amino acid may include a process of purifying the L-amino acid.

According to one embodiment of the present invention, the L-amino acid may be at least one selected from the group consisting of L-alanine, L-isoleucine, L-valine, L-leucine, L-methionine, L-asparagine, L-cysteine, L-glutamine, L-serine, L-threonine, L-phenylalanine, L-tryptophan, L-tyrosine, L-aspartic acid, L-glutamic acid, L-arginine, L-histidine, L-lysine, L-glycine, L-proline, and L-citrulline.

For example, the L-amino acid may be L-arginine, L-citrulline, L-lysine or L-glutamine, without being limited thereto.

### Advantageous Effects

The *Corynebacterium* sp. microorganism according to the present invention may have an increased L-amino acid production yield compared to a parent strain by having a weakened or inactivated activity of a carotenoid biosynthesis enzyme.

### Mode for Invention

Hereinafter, the present invention will be described in more detail. However, these descriptions are provided for illustrative purposes only to aid in the understanding of the present invention, and the scope of the present invention is not limited by these illustrative descriptions.

### Example 1. Construction of L-arginine-producing strains with disrupted carotenoid biosynthesis enzyme genes

To construct strains in which at least one of the Cgl0626, Cgl0624, Cgl0623, Cgl2433, Cgl2432 and Cgl2431 genes encoding carotenoid biosynthesis enzymes in an L-arginine-producing strain derived from *Corynebacterium glutamicum* has been disrupted, *Corynebacterium glutamicum* 14GR (KCCM13219P), which is an L-arginine-producing strain, and *E. coli* DH5a (HIT Competent cells^{™}, Cat No. RH618) were used.

The *Corynebacterium glutamicum* 14GR was cultured in an ARG-broth medium (pH 7.2) containing (per liter of distilled water) 10.5 g of 98% glucose, 1 g of beef extract, 4 g of yeast extract, 2 g of polypeptone, 2 g of NaCl and 40 g of (NH₄)₂SO₄ at a temperature of 30°C.

The *E. coli* DH5a was cultured on an LB medium containing 10.0 g of tryptone, 10.0 g of NaCl and 5.0 g of yeast extract in 1 L of distilled water at a temperature of 37°C.

The antibiotic kanamycin used was the product of Sigma.

DNA sequencing and gene synthesis were performed by Macrogen, Inc.

### 1-1. Recombinant vectors

Recombinant vectors for disrupting at least one of the genes Cgl0626, Cgl0624, Cgl0623, Cgl2433, Cgl2432, and Cgl2431 encoding carotenoid biosynthesis enzymes were constructed. The left arm (600-650-bp region) and the right arm (600-650-bp region) with respect to start and stop codons of each gene on the *Corynebacterium glutamicum* genome were amplified by PCR, ligated together by overlap PCR, and then cloned into the pk19mobsacB (ATCC, 87098) vector. To construct the plasmid, the primers shown in Table 1 below were used for amplification of each gene fragment.

**[Table 1]**

| Primer name | | Primer sequence (5'→3') | SEQ ID NO. |
|---|---|---|---|
| Cgl0626 and Cgl0624 disruption primers | LF1 | tgattacgccGCATCGCCTCTCCACGAGTG | 13 |
| | LF2 | GCATCGCCTCTCCACGAGTG | 14 |
| | LR1 | TCGTTGTATATGGCGCTTTA | 15 |
| | LR2 | AAATTCCCAATCGTTGTATA | 16 |
| | RF1 | TTGGGAATTTATGAAGGTCTCGACTAAAAC | 17 |
| | RF2 | ATGAAGGTCTCGACTAAAAC | 18 |
| | RR1 | GGAAGACAGGAAGACTGCTG | 19 |
| | RR2 | TAGTGGGTCGGGAAGACAGG | 20 |
| Cgl0623 disruption primers | LF1 | tgattacgccGCTTATGGTGAAACTGCCCG | 21 |
| | LF2 | GCTTATGGTGAAACTGCCCG | 22 |
| | LR1 | TTTCTGTAGATGCTCATAGA | 23 |
| | LR2 | tCACGAATTCTTTCTGTAGA | 24 |
| | RF1 | GAATTCGTGaATTTTGATCCCTATCATCGA | 25 |
| | RF2 | ATTTTGATCCCTATCATCGA | 26 |
| | RR1 | AGGTTATCAAATATGATAGT | 27 |
| | RR2 | CCAAACCATGAGGTTATCAA | 28 |
| Cgl2433 disruption primers | LF1 | tgattacgccGGGGACCGACCATCATCCAC | 29 |
| | LF2 | GGGGACCGACCATCATCCAC | 30 |
| | LR1 | ACCGCGAGAAATCCAGACAA | 31 |
| | LR2 | AGGAAACTTTACCGCGAGAA | 32 |
| | RF1 | AAAGTTTCCTGTGGAAAGAAGTGTTTCAAA | 33 |
| | RF2 | GTGGAAAGAAGTGTTTCAAA | 34 |
| | RR1 | GGGTAGCTTAAGATTTGGCG | 35 |
| | RR2 | GAAAACTGCGGGGTAGCTTA | 36 |
| Cgl2432 and Cgl2431 disruption primers | LF1 | tgattacgccTCAGTTTGTCCGTTCCTTTT | 37 |
| | LF2 | TCAGTTTGTCCGTTCCTTTT | 38 |
| | LR1 | AACACTTCTTTCcacATTGC | 39 |
| | LR2 | TCATTTTTGAAACACTTCTT | 40 |
| | RF1 | TCAAAAATGACCTGGAGCGGCGGGTCCATT | 41 |
| | RF2 | CCTGGAGCGGCGGGTCCATT | 42 |
| | RR1 | GAATGTTGGTGAACCATTTG | 43 |
| | RR2 | TCGGCTGCATGAATGTTGGT | 44 |

Using the above-described primers, PCR was performed under the following conditions. PCR was performed for 25 to 30 cycles using a Thermocycler (TP600, TAKARA BIO Inc., Japan) in a reaction solution containing 100 µM of each deoxynucleotide triphosphate (dATP, dCTP, dGTP, dTTP), 1 pM oligonucleotide, 10 ng of the chromosomal DNA of *Corynebacterium glutamicum* ATCC 13032 as a template, and 1 unit of pfu-X DNA polymerase mixture (Solgent). PCR was performed under the following conditions: (i) denaturation at 94°C for 30 sec, (ii) annealing at 58°C for 30 sec, and (iii) extension at 72°C for 1 to 2 min (a polymerization time of 2 min per kb).

The gene fragments produced as described above were cloned into the pk19mobsacB vector by self-assembly cloning. The vector was transformed into *E. coli* DH5a which was then plated on an LB-agar plate containing 50 µg/ml of kanamycin, and cultured at 37°C for 24 hours. The finally formed colony was isolated and whether the insert was exactly present in the vector was checked. Next, the vector was isolated and used for recombination of the *Corynebacterium glutamicum* strain.

As a process commonly performed in the above method, the genes of interest were amplified by PCR from the genomic DNA of *Corynebacterium glutamicum* ATCC 13032 and inserted into the pk19mobsacB vector by the self-assembled cloning method according to the strategy, and the resulting plasmid was selected in *E coli* DH5a. For chromosomal base substitution, the gene fragments were amplified individually and ligated together by overlap PCR, thereby producing the desired DNA fragment. For genetic manipulation, Ex Taq polymerase (Takara) and Pfu polymerase (Solgent) were used as PCR polymerases, and various restriction enzymes and DNA modifying enzymes purchased from NEB were used, and they were used according to manufacturer's provided buffers and protocols.

The Cgl0626 and Cgl0624 disruption recombinant vector constructed by the above-described method were named CD1, the Cgl0623 disruption recombinant vector was named CD2, the Cgl2433 disruption recombinant vector was named CD3, and the Cgl2432 and Cgl2431 disruption recombinant vector was named CD4.

### 1-2. Corynebacterium glutamicum mutant strains

Mutant strains were constructed using the cloning vectors CD1 to CD4. The strain constructed using CD1, which is the Cgl0626 and Cgl0624 disruption recombinant vector, was named DR1; the strain constructed using CD2, which is the Cgl0623 disruption recombinant vector, was named DR2; the strain constructed using CD3, which is the Cgl2433 disruption recombinant vector, was named DR3; and the strain constructed using CD4, which is the Cgl2432 and Cgl2431 disruption recombinant vector, was named DR4. The process for constructing each recombinant strain is as follows.

Each cloning vector was prepared at a final concentration of 1 µg/µl or more and electroporated into *Corynebacterium glutamicum* 14GR (see Tauch et al., FEMS Microbiology letters 123 (1994) 343-347) to induce first recombination. In this case, the electroporated strain was plated on an agar medium containing 50 µg/µl of kanamycin, colonies were isolated, and then whether the vector was properly inserted at the desired position on the genome was checked by PCR and sequencing. Each of the isolated strains was inoculated again in a liquid medium to induce second recombination, cultured overnight or more, and then plated on an agar medium containing 10% sucrose, and colonies were isolated. Whether the resulting isolated colonies were resistant to kanamycin was checked, and then whether the gene of interest in the strains having no antibiotic resistance would be disrupted was checked by sequencing (see Schafer et al., Gene 145 (1994) 69-73). Ultimately, L-arginine-producing *Corynebacterium glutamicum* mutant strains DR1, DR2, DR3 and DR4 with disruption of the genes encoding carotenoid biosynthesis enzymes were obtained.

### Experimental Example 1. Evaluation of L-arginine productivity of L-arginine-producing strains with disrupted carotenoid biosynthesis enzyme genes

The L-arginine productivity of each of the parent strain *Corynebacterium glutamicum* 14GR and the L-arginine-producin*g Corynebacterium glutamicum* DR1, DR2, DR3 and DR4 constructed in Example 1 was evaluated.

Each strain was patched on a flask solid seed medium and cultured at 30°C for 24 hours. Each cultured colony was inoculated into a 10-ml flask titer medium and cultured at 200 rpm at 32°C for 30 hours. The compositions of the media used here are shown in Table 2 below. After completion of the culturing, each culture was diluted 50-fold with distilled water and filtered through a 0.45-µm filter, and then the amount of L-arginine produced was analyzed using high-performance liquid chromatography (HPLC) (Agilent Technologies 1260 Infinity, Agilent Technologies) equipped with a column (DionexIonPac^{TM} CS12A) and a UV detector (195 mm), and the results are shown in Table 3 below.

**[Table 2]**

| | |
|---|---|
| Flask solid seed medium (per L) | 10.5 g of 98% glucose, 1 g of beef extract, 4 g of yeast extract, 2 g of polypeptone, 2 g of NaCl, 40 g of (NH₄)₂SO₄ and 20 g of agar |
| Flask titer medium (per L) | 120 g of 98% glucose, 1 g of MgSO₄, 2 g of KH₂PO₄, 45 g of (NH₄)₂SO₄, 20 mg of FeSO₄, 20 mg of MnSO₄, 100 µg of biotin, 100 µg of thiamine, 4 g of YSP and 2 g of urea |

**[Table 3]**

| Strain | OD₆₁₀ | L-arginine (%) | Yield (%) |
|---|---|---|---|
| 14GR | 40.0 | 2.1 | 26.4 |
| DR1 | 37.7 | 2.5 | 31.3 |
| DR2 | 38.0 | 2.4 | 30.0 |
| DR3 | 37.1 | 2.7 | 33.6 |
| DR4 | 38.2 | 2.4 | 30.0 |

As shown in Table 3 above, it was confirmed that *Corynebacterium glutamicum* DR1, DR2, DR3 and DR4 with disruption of the genes encoding carotenoid biosynthesis enzymes showed an increase in L-arginine production of about 14.3 to 28.6% and an increase in L-arginine production yield of about 3.6 to 7.2%p, compared to the parent strain *Corynebacterium glutamicum* 14GR.

### Example 2. Construction of L-citrulline-producing strains with disrupted carotenoid biosynthesis enzyme genes

To construct strains in which at least one of the Cgl0626, Cgl0624, Cgl0623, Cgl2433, Cgl2432 and Cgl2431 genes encoding carotenoid biosynthesis enzymes in an L-citrulline-producing strain derived from *Corynebacterium glutamicum* has been disrupted, *Corynebacterium glutamicum* CT4, which is a L-citrulline-producing strain, and *E. coli* DH5a (HIT Competent cells^{™}, Cat No. RH618) were used.

The *Corynebacterium glutamicum* CT4 is a L-citrulline-producing strain in which the activity of ornithine carbamoyltransferase and carbamoyl phosphate synthetase has been enhanced to produce an excessive amount of citrulline (see Korean Patent Application No. 10-2019-0151321). The strain was cultured on a medium containing (per liter of distilled water) 10.5% glucose, 1 g of beef extract, 4 g of yeast extract, 2 g of polypeptone, 2 g of NaCl, 40 g of (NH₄)₂SO₄ and 20 g of agar at a temperature of 30°C.

In addition, culture conditions for *E. coli* DH5a and methods for constructing recombinant vectors CD1 to CD4 are as described in Example 1-1.

Mutant strains were constructed using the constructed cloning vectors CD1 to CD4. The strain constructed using CD1, which is the Cgl0626 and Cgl0624 disruption recombinant vector, was named DC1; the strain constructed using CD2, which is the Cgl0623 disruption recombinant vector, was named DC2; the strain constructed using CD3, which is the Cgl2433 disruption recombinant vector, was named DC3; and the strain constructed using CD4, which is the Cgl2432 and Cgl2431 disruption recombinant vector, was named DC4. The process for constructing each recombinant strain is as follows.

Each cloning vector was prepared at a final concentration of 1 µg/µl or more and electroporated into *Corynebacterium glutamicum* CT4 (see Tauch et al., FEMS Microbiology letters 123 (1994) 343-347) to induce first recombination. In this case, the electroporated strain was plated on an agar medium containing 50 µg/µl of kanamycin, colonies were isolated, and then whether the vector was properly inserted at the desired position on the genome was checked by PCR and sequencing. Each of the isolated strains was inoculated again in a liquid medium to induce second recombination, cultured overnight or more, and then plated on an agar medium containing 10% sucrose, and colonies were isolated. Whether the resulting isolated colonies were resistant to kanamycin was checked, and then whether the gene of interest in the strains having no antibiotic resistance would be disrupted was checked by sequencing (see Schafer et al., Gene 145 (1994) 69-73). Ultimately, L-citrulline-producing *Corynebacterium glutamicum* mutant strains DC1, DC2, DC3 and DC4 with disruption of the genes encoding carotenoid biosynthesis enzymes were obtained.

### Experimental Example 2. Evaluation of L-citrulline productivity of L-citrulline-producing strains with disrupted carotenoid biosynthesis enzyme genes

The L-citrulline productivity of each of the parent strain *Corynebacterium glutamicum* CT4 and the L-citrulline-producin*g Corynebacterium glutamicum* DC1, DC2, DC3 and DC4 constructed in Example 2 was evaluated.

Each strain was patched on a flask solid seed medium and cultured at 30°C for 24 hours. Each cultured colony was inoculated into a 10-ml flask titer medium and cultured at 180 rpm at 30°C for 30 hours. The compositions of the media used here are shown in Table 4 below. After completion of the culturing, each culture was diluted 50-fold with distilled water and filtered through a 0.45-µm filter, and then the amount of L-citrulline produced was analyzed using high-performance liquid chromatography (HPLC) (Agilent Technologies 1260 Infinity, Agilent Technologies) equipped with a column (DionexIonPac^{TM} CS12A) and a UV detector (195 mm), and the results are shown in Table 5 below.

**[Table 4]**

| | |
|---|---|
| Flask solid seed medium (per L) | 10.5 g of 98% glucose, 1 g of beef extract, 4 g of yeast extract, 2 g of polypeptone, 2 g of NaCl, 40 g of (NH₄)₂SO₄ and 20 g of agar |
| Flask titer medium (per L) | 105.3 g of glucose (95%), 1 g of MgSO₄, 4 g of YPA, 0.8 g of KH₂PO₄, 1.2 g of Na₂HPO₄, 30 g of (NH₄)₂SO₄, 20 mg of Fe_{S}O₄, 20 mg of MnSO₄, 10 mg of ZnSO₄, 100 mg of arginine, 100 µg of biotin, and 200 µg of thiamine |

**[Table 5]**

| Strain | OD₆₁₀ | L-citrulline (%) | Yield (%) |
|---|---|---|---|
| CT4 | 35.1 | 1.2 | 12.2 |
| DC1 | 33.5 | 1.6 | 16.2 |
| DC2 | 33.9 | 1.5 | 14.8 |
| DC3 | 33.2 | 1.7 | 17.2 |
| DC4 | 33.8 | 1.5 | 15.2 |

As shown in Table 5 above, it was confirmed that *Corynebacterium glutamicum* DC1, DC2, DC3 and DC4 with disruption of the genes encoding carotenoid biosynthesis enzymes showed an increase in L-citrullne production of about 25.0 to 41.7% and an increase in L-citrulline production yield of about 2.6 to 5.0%p, compared to the parent strain *Corynebacterium glutamicum* CT4.

### Example 3. Construction of L-lysine-producing strains with disrupted carotenoid biosynthesis enzyme genes

To construct strains in which at least one of the Cgl0626, Cgl0624, Cgl0623, Cgl2433, Cgl2432 and Cgl2431 genes encoding carotenoid biosynthesis enzymes in an L-lysine-producing strain derived from *Corynebacterium glutamicum* has been disrupted, *Corynebacterium glutamicum* DS1 (KCCM12969P), which is a L-lysine-producing strain, and *E. coli* DH5a (HIT Competent cells^{™}, Cgl2433, Cg618) were used.

The *Corynebacterium glutamicum* DS1 was cultured on a medium (pH 7.2) containing (per liter of distilled water) 5 g of 98% glucose, 5 g of beef extract, 4 g of yeast extract, 5 g of polypeptone, 2 g of NaCl, 40 g of (NH₄)₂SO₄ and 20 g of agar at a temperature of 30°C.

In addition, culture conditions for *E. coli* DH5a and methods for constructing recombinant vectors CD1 to CD4 are as described in Example 1-1.

Mutant strains were constructed using the constructed cloning vectors CD1 to CD4. The strain constructed using CD1, which is the Cgl0626 and Cgl0624 disruption recombinant vector, was named DK1; the strain constructed using CD2, which is the Cgl0623 disruption recombinant vector, was named DK2; the strain constructed using CD3, which is the Cgl2433 disruption recombinant vector, was named DK3; and the strain constructed using CD4, which is the Cgl2432 and Cgl2431 disruption recombinant vector, was named DK4. The process for constructing each recombinant strain is as follows.

Each cloning vector was prepared at a final concentration of 1 µg/µl or more and electroporated into *Corynebacterium glutamicum* DS1 (see Tauch et al., FEMS Microbiology letters 123 (1994) 343-347) to induce first recombination. In this case, the electroporated strain was plated on an agar medium containing 50 µg/µl of kanamycin, colonies were isolated, and then whether the vector was properly inserted at the desired position on the genome was checked by PCR and sequencing. Each of the isolated strains was inoculated again in a liquid medium to induce second recombination, cultured overnight or more, and then plated on an agar medium containing 10% sucrose, and colonies were isolated. Whether the resulting isolated colonies were resistant to kanamycin was checked, and then whether the gene of interest in the strains having no antibiotic resistance would be disrupted was checked by sequencing (see Schafer et al., Gene 145 (1994) 69-73). Ultimately, L-lysine-producing *Corynebacterium glutamicum* mutant strains DK1, DK2, DK3 and DK4 with disruption of the genes encoding carotenoid biosynthesis enzymes were obtained.

### Experimental Example 3. Evaluation of L-lysine productivity of L-lysine-producing strains with disrupted carotenoid biosynthesis enzyme genes

The L-lysine productivity of each of the parent strain *Corynebacterium glutamicum* DS1 and the L-lysine-producin*g Corynebacterium glutamicum* DK1, DK2, DK3 and DK4 constructed in Example 3 was evaluated.

Each strain was patched on a flask solid seed medium and cultured at 30°C for 24 hours. Each cultured colony was inoculated into a 10-ml flask titer medium and cultured at 180 rpm at 30°C for 28 hours. The compositions of the media used here are shown in Table 6 below. After completion of the culturing, each culture was diluted 10-fold with distilled water and filtered through a 0.45-µm filter, and then the amount of L-lysine produced was analyzed using high-performance liquid chromatography (HPLC) (Agilent Technologies 1260 Infinity, Agilent Technologies) equipped with a column (DionexIonPac^{TM} CS12A) and a UV detector (195 mm), and the results are shown in Table 7 below.

**[Table 6]**

| | |
|---|---|
| Flask solid seed medium (per L) | 5 g of 98% glucose, 5 g of beef extract, 4 g of yeast extract, 5 g of polypeptone, 2 g of NaCl, 40 g of (NH₄)₂SO₄, and 20 g of agar |
| Flask titer medium (per L) | 100 g of 98% glucose, 1 g of MgSO₄, 1 g of KH₂PO₄, 55 g of (NH₄)₂SO₄, 20 mg of FeSO₄, 20 mg of MnSO₄, 100 µg of biotin, 100 µg of thiamine, 4 g of YSP, and 2 g of urea |

**[Table 7]**

| Strain | OD₆₁₀ | L-lysine (%) | Yield (%) |
|---|---|---|---|
| DS1 | 32 | 6.0 | 60.0 |
| DK1 | 30 | 6.6 | 66.6 |
| DK2 | 30 | 6.4 | 64.1 |
| DK3 | 28 | 6.7 | 67.1 |
| DK4 | 31 | 6.4 | 64.0 |

As shown in Table 7 above, it was confirmed that *Corynebacterium glutamicum* DK1, DK2, DK3 and DK4 with disruption of the genes encoding carotenoid biosynthesis enzymes showed an increase in L-lysine production of about 6.7 to 11.7% and an increase in L-lysine production yield of 4.0 to 7.1%p, compared to the parent strain *Corynebacterium glutamicum* DS1.

### Example 4. Construction of L-glutamine-producing strains with disrupted carotenoid biosynthesis enzyme genes

To construct strains in which at least one of the Cgl0626, Cgl0624, Cgl0623, Cgl2433, Cgl2432 and Cgl2431 genes encoding carotenoid biosynthesis enzymes in an L-glutamine-producing strain derived from *Corynebacterium glutamicum* has been disrupted, *Corynebacterium glutamicum* DQ3-6, which is a L-glutamine-producing strain (KCCM13398P), and *E. coli* DH5a (HIT Competent cells^{™}, Cat No. RH618) were used.

The *Corynebacterium glutamicum* DQ3-6 was cultured in a broth medium (pH 7.2) containing (per liter of distilled water) 10.5 g of 98% glucose, 1 g of beef extract, 4 g of yeast extract, 2 g of polypeptone, 2 g of NaCl, 5 g of urea, 50 mg of alanine, and 40 g of (NH₄)₂SO₄ at a temperature of 30°C.

In addition, culture conditions for *E. coli* DH5a and methods for constructing recombinant vectors CD1 to CD4 are as described in Example 1-1.

Mutant strains were constructed using the constructed cloning vectors CD1 to CD4. The strain constructed using CD1, which is the Cgl0626 and Cgl0624 disruption recombinant vector, was named DE1; the strain constructed using CD2, which is the Cgl0623 disruption recombinant vector, was named DE2; the strain constructed using CD3, which is the Cgl2433 disruption recombinant vector, was named DE3; and the strain constructed using CD4, which is the Cgl2432 and Cgl2431 disruption recombinant vector, was named DE4. The process for constructing each recombinant strain is as follows.

Each cloning vector was prepared at a final concentration of 1 µg/µl or more and electroporated into *Corynebacterium glutamicum* DQ3-6 (see Tauch et al., FEMS Microbiology letters 123 (1994) 343-347) to induce first recombination. In this case, the electroporated strain was plated on an agar medium containing 50 µg/µl of kanamycin, colonies were isolated, and then whether the vector was properly inserted at the desired position on the genome was checked by PCR and sequencing. Each of the isolated strains was inoculated again in a liquid medium to induce second recombination, cultured overnight or more, and then plated on an agar medium containing 10% sucrose, and colonies were isolated. Whether the resulting isolated colonies were resistant to kanamycin was checked, and then whether the gene of interest in the strains having no antibiotic resistance would be disrupted was checked by sequencing (see Schafer et al., Gene 145 (1994) 69-73). Ultimately, L-glutamine-producing *Corynebacterium glutamicum* mutant strains DE1, DE2, DE3 and DE4 with disruption of the genes encoding carotenoid biosynthesis enzymes were obtained.

### Experimental Example 4. Evaluation of L-glutamine productivity of L-glutamine-producing strains with disrupted carotenoid biosynthesis enzyme genes

The L-glutamine productivity of each of the parent strain *Corynebacterium glutamicum* DQ3-6 and the L-glutamine-producing *Corynebacterium glutamicum* DE1, DE2, DE3 and DE4 constructed in Example 4 was evaluated.

Each strain was patched on a flask solid seed medium and cultured at 30°C for 24 hours. Each cultured colony was inoculated into a 10-ml flask titer medium and cultured at 160 rpm at 30°C for 72 hours. The compositions of the media used here are shown in Table 8 below. After completion of the culturing, each culture was diluted 100-fold with distilled water and filtered through a 0.45-µm filter, and then the amount of L-glutamine produced was analyzed using high-performance liquid chromatography (HPLC) (Agilent Technologies 1260 Infinity, Agilent Technologies) equipped with a column (DionexIonPac^{TM} CS12A) and a UV detector (195 mm), and the results are shown in Table 9 below.

**[Table 8]**

| | |
|---|---|
| Flask solid seed medium (per L) | 5 g of 98% glucose, 1 g of beef extract, 4 g of yeast extract, 2 g of polypeptone, 2 g of NaCl, 40 g of (NH₄)₂SO₄, and 20 g of agar |
| Flask titer medium (per L) | 100 g of 98% glucose, 1 g of MgSO₄, 2 g of KH₂PO₄, 45 g of (NH₄)₂SO₄, 20 mg of FeSO₄, 20 mg of MnSO₄, 100 µg of biotin, 100 µg of thiamine, 4 g of YSP, 2.0 g of urea, 50 mg of L-alanine, and 50 mg of L-cystein |

**[Table 9]**

| Strain | OD₆₁₀ | L-glutamine (%) | Yield (%) |
|---|---|---|---|
| DQ3-6 | 25.6 | 2.9 | 29.4 |
| DE1 | 24.3 | 3.3 | 32.8 |
| DE2 | 25.1 | 3.3 | 32.6 |
| DE3 | 24.2 | 3.4 | 33.5 |
| DE4 | 24.8 | 3.5 | 32.6 |

As shown in Table 9 above, it was confirmed that *Corynebacterium glutamicum* DE1, DE2, DE3 and DE4 with disruption of the genes encoding carotenoid biosynthesis enzymes showed an increase in L-glutamine production of about 13.8 to 20.7% and an increase in L-glutamine production yield of about 3.2 to 4.1%p, compared to the parent strain *Corynebacterium glutamicum* DQ3-6.

Taking the above results together, it can be seen that, by weakening or inactivating the activity of the carotenoid biosynthetic enzyme encoded by the crtB gene, the crtB2 gene, or the cluster of the crtB/crtB2 genes in a *Corynebacterium* sp. microorganism, the production and yield of L-amino acids, including L-arginine, L-citrulline, L-lysine, and L-glutamine, may be improved compared to those in the microorganism before modification.

So far, the present invention has been described with reference to the preferred embodiments. Those of ordinary skill in the art to which the present invention pertains will appreciate that the present invention may be embodied in modified forms without departing from the essential characteristics of the present invention. Therefore, the disclosed embodiments should be considered from an illustrative point of view, not from a restrictive point of view. The scope of the present invention is defined by the claims rather than the foregoing description, and all differences within the scope equivalent thereto should be construed as being included in the present invention.

### [Depository authority]

Depository authority: Korean Culture Center of Microorganisms (KCCM)
Accession number: KCCM13219P
Deposit date: June 29, 2022
Depository authority: Korean Culture Center of Microorganisms (KCCM)
Accession number: KCCM12969P
Deposit date: April 2, 2021
Depository authority: Korean Culture Center of Microorganisms (KCCM)
Accession number: KCCM13398P
Deposit date: September 26, 2023

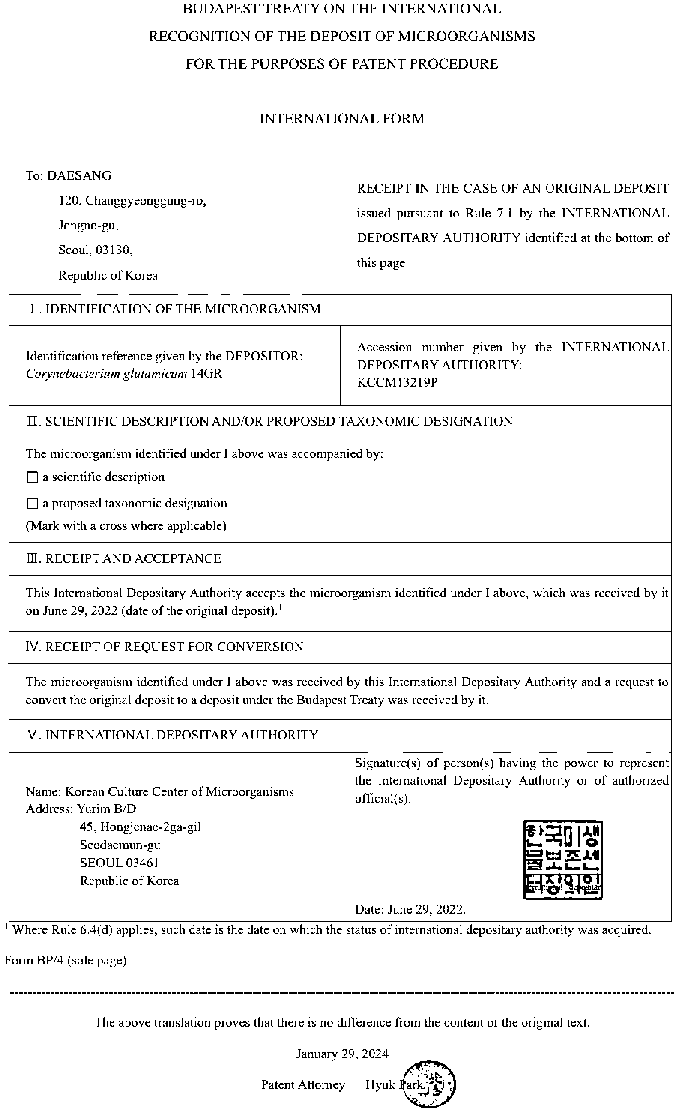
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To: DAESANG
120, Changgyeonggung-ro,
Jongno-gu,
Seoul, 03130,
Republic of Korea

RECEIPT IN THE CASE OF AN ORIGINAL DEPOSIT
issued pursuant to Rule 7.1 by the INTERNATIONAL
DEPOSITARY AUTHORITY identified at the bottom of
this page

I. IDENTIFICATION OF THE MICROORGANISM
Identification reference given by the DEPOSITOR:
Corynebacterium glutamicum 14GR | Accession number given by the INTERNATIONAL
DEPOSITARY AUTHORITY:
KCCM13219P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above, which was received by it
on June 29, 2022 (date of the original deposit).¹
IV. RECEIPT OF REQUEST FOR CONVERSION
The microorganism identified under I above was received by this International Depositary Authority and a request to
convert the original deposit to a deposit under the Budapest Treaty was received by it.
V. INTERNATIONAL DEPOSITARY AUTHORITY
Name: Korean Culture Center of Microorganisms
Address: Yurim B/D
45, Hongjenae-2ga-gil
Seodaemun-gu
SEOUL 03461
Republic of Korea | Signature(s) of person(s) having the power to represent
the International Depositary Authority or of authorized
official(s):
Date: June 29, 2022.
¹ Where Rule 6.4(d) applies, such date is the date on which the status of international depositary authority was acquired.

Form BP/4 (sole page)

The above translation proves that there is no difference from the content of the original text.

January 29, 2024

Patent Attorney Hyuk Park

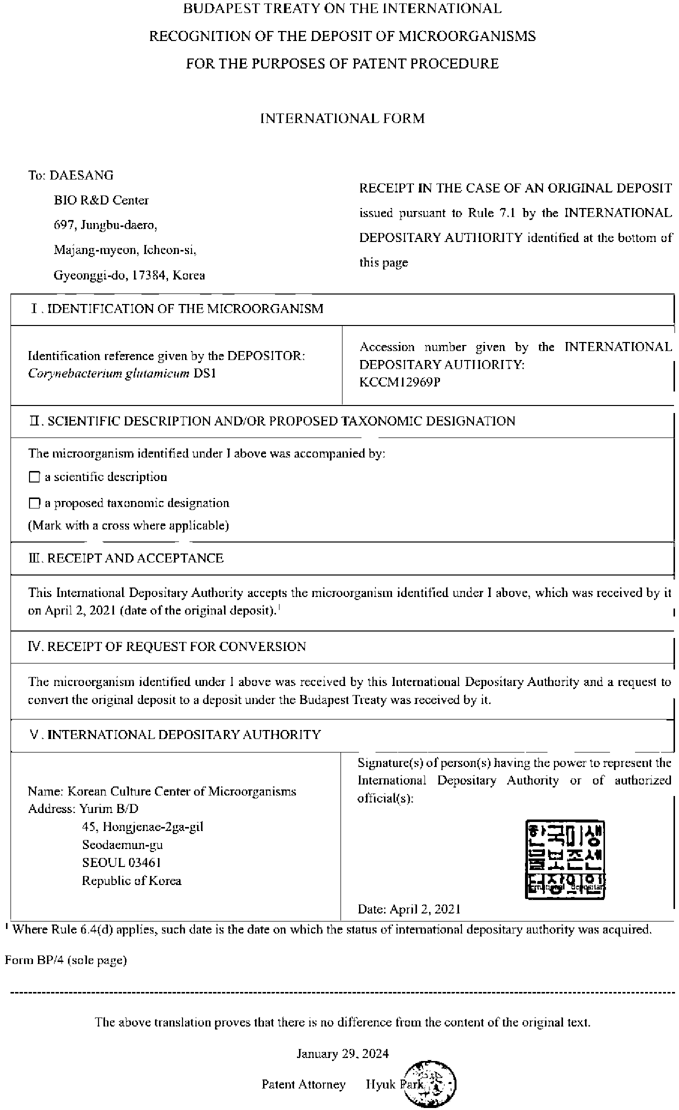
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To: DAESANG
BIO R&D Center
697, Jungbu-daero,
Majang-myeon, Icheon-si,
Gyeonggi-do, 17384, Korea

RECEIPT IN THE CASE OF AN ORIGINAL DEPOSIT
issued pursuant to Rule 7.1 by the INTERNATIONAL
DEPOSITARY AUTHORITY identified at the bottom of
this page

I . IDENTIFICATION OF THE MICROORGANISM
Identification reference given by the DEPOSITOR:
Corynebacterium glutamicum DS1 | Accession number given by the INTERNATIONAL
DEPOSITARY AUTHORITY:
KCCM12969P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above, which was received by it
on April 2, 2021 (date of the original deposit).¹
IV. RECEIPT OF REQUEST FOR CONVERSION
The microorganism identified under I above was received by this International Depositary Authority and a request to
convert the original deposit to a deposit under the Budapest Treaty was received by it.
V. INTERNATIONAL DEPOSITARY AUTHORITY
Name: Korean Culture Center of Microorganisms
Address: Yurim B/D
45, Hongjenae-2ga-gil
Seodaemun-gu
SEOUL 03461
Republic of Korea | Signature(s) of person(s) having the power to represent the
International Depositary Authority or of authorized
official(s):
Date: April 2, 2021
¹ Where Rule 6.4(d) applies, such date is the date on which the status of international depositary authority was acquired.

Form BP/4 (sole page)

--------------------------------------------------------------------------------------------------------------------------------

The above translation proves that there is no difference from the content of the original text.

January 29, 2024

Patent Attorney Hyuk Park

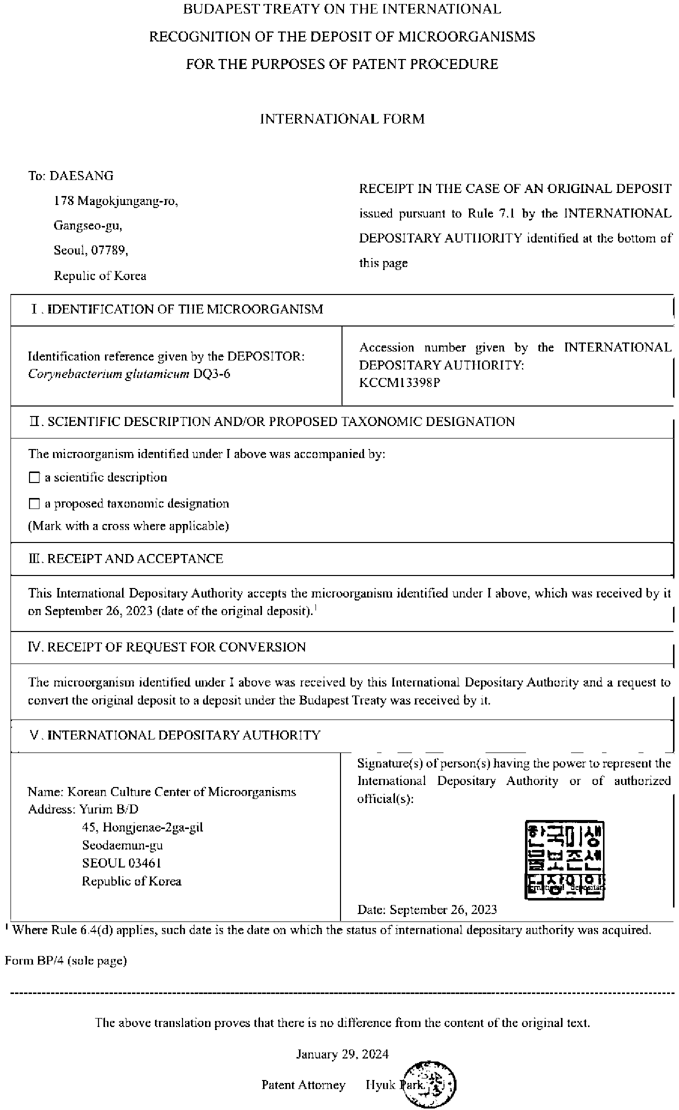
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To: DAESANG
178 Magokjungang-ro,
Gangseo-gu,
Seoul, 07789,
Repulic of Korea

RECEIPT IN THE CASE OF AN ORIGINAL DEPOSIT
issued pursuant to Rule 7.1 by the INTERNATIONAL
DEPOSITARY AUTHORITY identified at the bottom of
this page

I . IDENTIFICATION OF THE MICROORGANISM
Identification reference given by the DEPOSITOR:
Corynebacterium glutamicum DQ3-6 | Accession number given by the INTERNATIONAL
DEPOSITARY AUTHORITY:
KCCM13398P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above, which was received by it
on September 26, 2023 (date of the original deposit).¹
IV. RECEIPT OF REQUEST FOR CONVERSION
The microorganism identified under I above was received by this International Depositary Authority and a request to
convert the original deposit to a deposit under the Budapest Treaty was received by it.
V. INTERNATIONAL DEPOSITARY AUTHORITY
Name: Korean Culture Center of Microorganisms
Address: Yurim B/D
45, Hongjenae-2ga-gil
Seodaemun-gu
SEOUL 03461
Republic of Korea | Signature(s) of person(s) having the power to represent the
International Depositary Authority or of authorized
official(s):
Date: September 26, 2023
¹ Where Rule 6.4(d) applies, such date is the date on which the status of international depositary authority was acquired.

Form BP/4 (sole page)

The above translation proves that there is no difference from the content of the original text.

January 29, 2024

Patent Attorney Hyuk Park.

## Claims

1. A *Corynebacterium* sp. microorganism having improved L-amino acid productivity having a weakened or inactivated activity of a carotenoid biosynthesis enzyme.

2. The *Corynebacterium* sp. microorganism of claim 1, wherein weakening of the activity of the carotenoid biosynthesis enzyme is achieved by insertion, substitution, deletion, or a combination thereof of all or part of a gene encoding the carotenoid biosynthesis enzyme.

3. The *Corynebacterium* sp. microorganism of claim 2, wherein the gene encoding the carotenoid biosynthesis enzyme is at least one selected from the group consisting of Cgl0626, Cgl0624, Cgl0623, Cgl2433, Cgl2432, and Cgl2431.

4. The *Corynebacterium* sp. microorganism of claim 3, wherein
the Cgl0626 gene comprises the nucleotide sequence of SEQ ID NO: 1,
the Cgl0624 gene comprises the nucleotide sequence of SEQ ID NO: 3,
the Cgl0623 gene comprises the nucleotide sequence of SEQ ID NO: 5,
the Cgl2433 gene comprises the nucleotide sequence of SEQ ID NO: 7,
the Cgl2432 gene comprises the nucleotide sequence of SEQ ID NO: 9, and
the Cgl2431 gene comprises the nucleotide sequence of SEQ ID NO: 11.

5. The *Corynebacterium* sp. microorganism of claim 1, wherein the *Corynebacterium* sp. microorganism is *Corynebacterium glutamicum*.

6. The *Corynebacterium* sp. microorganism of claim 1, wherein the L-amino acid is at least one selected from the group consisting of L-alanine, L-isoleucine, L-valine, L-leucine, L-methionine, L-asparagine, L-cysteine, L-glutamine, L-serine, L-threonine, L-phenylalanine, L-tryptophan, L-tyrosine, L-aspartic acid, L-glutamic acid, L-arginine, L-histidine, L-lysine, L-glycine, L-proline, and L-citrulline.

7. A method for producing L-amino acid, comprising steps of:
culturing the *Corynebacterium* sp. microorganism of claim 1 in a medium; and
recovering L-amino acid from the microorganism or the medium in which the microorganism has been cultured.

8. The method of claim 7, wherein the L-amino acid is at least one selected from the group consisting of L-alanine, L-isoleucine, L-valine, L-leucine, L-methionine, L-asparagine, L-cysteine, L-glutamine, L-serine, L-threonine, L-phenylalanine, L-tryptophan, L-tyrosine, L-aspartic acid, L-glutamic acid, L-arginine, L-histidine, L-lysine, L-glycine, L-proline, and L-citrulline.
